# EUROPEAN PATENT SPECIFICATION

(11) **EP 1 558 278 B1**
(45) Date of publication and mention of the grant of the patent: **02.09.2009**
(21) Application number: 03776440.4
(22) Date of filing: 10.10.2003
(51) Int. Cl.: A61K 38/17, C07K 14/705, C07K 14/715, C12N 5/10, C12N 15/12

(54) **PRODUCTION OF HOMOTRIMERIC FUSION PROTEINS**
HERSTELLUNG VON HOMOTRIMEREN FUSIONSPROTEINEN
PRODUCTION DE PROTEINES HYBRIDES HOMO-TRIMERIQUES

(30) Priority: 11.10.2002 US 417801 P
(43) Date of publication of application: 03.08.2005
(73) Proprietor: ZymoGenetics, Inc., Seattle, WA 98102 (US)
(72) Inventor: WEST, James, W., Santa Barbara, CA 93105 (US); BRANDT, Cameron, S., Seattle, WA 98115 (US); JASPERS, Stephen, R., Edmonds, WA 98026 (US)
(74) Representative: MacLean, Martin Robert
(86) International application number: PCT/US2003/032878
(87) International publication number: WO 2004/033486

(56) References cited:
- WO-A-98/39361
- US-A1- 2002 086 018
- US-B1- 6 218 513
- US-B1- 6 534 061
- GROSS J A ET AL: "TACI and BCMA are receptors for a TNF homologue implicated in B-cell autoimmune disease" NATURE, NATURE PUBLISHING GROUP, LONDON, GB, vol. 404, 27 April 2000 (2000-04-27), pages 995-999, XP002140939 ISSN: 0028-0836
- TAI LI-JUNG ET AL: "Structure-function analysis of the heat shock factor-binding protein reveals a protein composed solely of a highly conserved and dynamic coiled-coil trimerization domain." THE JOURNAL OF BIOLOGICAL CHEMISTRY 4 JAN 2002, vol. 277, no. 1, 4 January 2002 (2002-01-04), pages 735-745, XP002457300 ISSN: 0021-9258
- FRISCHHOLZ S ET AL: "Characterization of human type X procollagen and its NC-1 domain expressed as recombinant proteins in HEK293 cells." THE JOURNAL OF BIOLOGICAL CHEMISTRY 20 FEB 1998, vol. 273, no. 8, 20 February 1998 (1998-02-20), pages 4547-4555, XP002457301 ISSN: 0021-9258

### SEQUENCE LISTING

<110> ZymoGenetics, Inc.
<120> Production of Homotrimeric Fusion Proteins
<130> 02-17PC
<150> 60/417,801
   <151> 2002-10-11
<160> 22
<170> FastSEQ for Windows Version 3.0
<210> 1
   <211> 10
   <212> PRT
   <213> Artificial Sequence
<220>
   <223> C-myc tag.
<400> 1
   Glu Gln Lys Leu Ile Ser Glu Glu Asp Leu
    1               5                  10
<210> 2
   <211> 9
   <212> PRT
   <213> Artificial Sequence
<220>
   <223> Hemagglutinin A epitope tag
<400> 2
   Tyr Pro Tyr Asp Val Pro Asp Tyr Ala
    1               5
<210> 3
   <211> 1377
   <212> DNA
   <213> Human
<220>
   <221> CDS
   <222> (14)...(892)
<400> 3
agcatcctga gta atg agt ggc ctg ggc cgg agc agg cga ggt ggc cgg        49
   agc cgt gtg gac cag gag gag cgc ttt cca cag ggc ctg tgg acg ggg        97
   gtg gct atg aga tcc tgc ccc gaa gag cag tac tgg gat cct ctg ctg       145
   ggt acc tgc atg tcc tgc aaa acc att tgc aac cat cag agc cag cgc      193
   acc tgt gca gcc ttc tgc agg tca ctc agc tgc cgc aag gag caa ggc      241
   aag ttc tat gac cat ctc ctg agg gac tgc atc agc tgt gcc tcc atc      289
   tgt gga cag cac cct aag caa tgt gca tac ttc tgt gag aac aag ctc      337
   agg agc cca gtg aac ctt cca cca gag ctc agg aga cag cgg agt gga      385
   gaa gtt gaa aac aat tca gac aac tcg gga agg tac caa gga ttg gag      433
   cac aga ggc tca gaa gca agt cca gct ctc ccg ggg ctg aag ctg agt      481
   gca gat cag gtg gcc ctg gtc tac agc acg ctg ggg ctc tgc ctg tgt      529
   gcc gtc ctc tgc tgc ttc ctg gtg gcg gtg gcc tgc ttc ctc aag aag      577
   agg ggg gat ccc tgc tcc tgc cag ccc cgc tca agg ccc cgt caa agt      625
   ccg gcc aag tct tcc cag gat cac gcg atg gaa gcc ggc agc cct gtg      673
   agc aca tcc ccc gag cca gtg gag acc tgc agc ttc tgc ttc cct gag      721
   tgc agg gcg ccc acg cag gag agc gca gtc acg cct ggg acc ccc gac      769
   ccc act tgt gct gga agg tgg ggg tgc cac acc agg acc aca gtc ctg      817
   cag cct tgc cca cac atc cca gac agt ggc ctt ggc att gtg tgt gtg      865
   cct gcc cag gag ggg ggc cca ggt gca taaatggggg tcagggaggg            912
   aaaggaggag ggagagagat ggagaggagg ggagagagaa agagaggtgg ggagagggga    972
   gagagatatg aggagagaga gacagaggag gcagaaaggg agagaaacag aggagacaga   1032
   gagggagaga gagacagagg gagagagaga cagaggggaa gagaggcaga gagggaaaga   1092
   ggcagagaag gaaagagaca ggcagagaag gagagaggca gagagggaga gaggcagaga   1152
   gggagagagg cagagagaca gagagggaga gagggacaga gagagataga gcaggaggtc   1212
   ggggcactct gagtcccagt tcccagtgca gctgtaggtc gtcatcacct aaccacacgt   1272
   gcaataaagt cctcgtgcct gctgctcaca gcccccgaga gcccctcctc ctggagaata   1332
   aaacctttgg cagctgccct tcctcaaaaa aaaaaaaaaa aaaaa                   1377
<210> 4
   <211> 293
   <212> PRT
   <213> Human
<400> 4
<210> 5
   <211> 21
   <212> DNA
   <213> Artificial Sequence
<220>
   <223> PCR primer
<400> 5
   gggcctccag gcccaccagg t 21
<210> 6
   <211> 21
   <212> DNA
   <213> Artificial Sequence
<220>
   <223> PCR primer
<400> 6
   tcacattgga gccactagga a 21
<210> 7
   <211> 56
   <212> DNA
   <213> Artificial Sequence
<220>
   <223> PCR primer
<400> 7
   acaggtgtcc agggaattca tataggccgg ccaccatgga tgcaatgaag agaggg 56
<210> 8
   <211> 36
   <212> DNA
   <213> Artificial Sequence
<220>
   <223> PCR primer
<400> 8
   accctcaggc atcgaacccg aacccgaacc ggatcc 36
<210> 9
   <211> 118
   <212> DNA
   <213> Artificial Sequence
<220>
   <223> PCR primer
<400> 9
   gatcggatcc atggccgaaa ctgatcctaa aacagttcaa gaccttacca gcgtagtcca 60
   gacgctcctg caagagatcg aagataagtt tcagactatg agcgaccaaa tcattgag 118
<210> 10
   <211> 100
   <212> DNA
   <213> Artificial Sequence
<220>
   <223> PCR primer
<400> 10
   agaatgcatg acatgagctc caggatagat gaccttgaga aaaatatagc agatttaatg 60
   acgcaagctg gtgtggaaga gttggaagga agtggttcta 100
<210> 11
   <211> 110
   <212> DNA
   <213> Artificial Sequence
<220>
   <223> PCR primer
<400> 11
   gatctagaac cacttccttc caactcttcc acaccagctt gcgtcattaa atctgctata 60
   tttttctcaa ggtcatctat cctggagctc atgtcatcga ttctctcaat 110
<210> 12
   <211> 108
   <212> DNA
   <213> Artificial Sequence
<220>
   <223> PCR primer
<400> 12
   gatttggtcg ctcatagtct gaaacttatc ttgcatctct tgcaggagcg tctggactac 60
   gctggtaagg tcttgaactg ttttaggatc agtttcggcc atggatcc 108
<210> 13
   <211> 33
   <212> DNA
   <213> Artificial Sequence
<220>
   <223> PCR primer
<400> 13
   cacacgtacg aagatggatg caatgaagag agg 33
<210> 14
   <211> 30
   <212> DNA
   <213> Artificial Sequence
<220>
   <223> PCR primer
<400> 14
   ggttagatct cgaacccgaa cccgaaccgg 30
<210> 15
   <211> 62
   <212> DNA
   <213> Artificial Sequence
<220>
   <223> PCR primer
<400> 15
   ctagaaataa ttttgtttaa ctttaagaag gagatatata tatggctatg agatcctgcc     60
   cc                                                                    62
<210> 16
   <211> 64
   <212> DNA
   <213> Artificial Sequence
<220>
   <223> PCR primer
<400> 16
   tctgtatcag gctgaaaatc ttatctcatc cgccaaaaca ctagtgatgg tgatggtgat     60
   ggcc                                                                  64
<210> 17
   <211> 516
   <212> DNA
   <213> Artificial Sequence
<220>
   <223> TACI-FiSBP fragment
<400> 17
   atggctatga gatcctgccc cgaagagcag tactgggatc ctctgctggg tacctgcatg     60
   tcctgcaaaa ccatttgcaa ccatcagagc cagcgcacct gtgcagcctt ctgcaggtca    120
   ctcagctgcc gcaaggagca aggcaagttc tatgaccatc tcctgaggga ctgcatcagc    180
   tgtgcctcca tctgtggaca gcaccctaag caatgtgcat acttctgtga gaacaagctc    240
   aggagcggat ccggttcggg ttcgggttcg agatccatgg ccgaaactga tcctaaaaca    300
   gttcaagacc ttaccagcgt agtccagacg ctcctgcaag agatgcaaga taagtttcag    360
   actatgagcg accaaatcat tgagagaatc gatgacatga gctccaggat agatgacctt    420
   gagaaaaata tagcagattt aatgacgcaa gctggtgtgg aagagttgga aggaagtggt    480
   tctagatccg gtggccatca ccatcaccat cactga                              516
<210> 18
   <211> 171
   <212> PRT
   <213> Artificial Sequence
<220>
   <223> TACI-HSBP fragment
<400> 18
   Met Ala Met Arg Ser Cys Pro Glu Glu Gln Tyr Trp Asp Pro Leu Leu
    1               5                  10                  15
   Gly Thr Cys Met Ser Cys Lys Thr Ile Cys Asn His Gln Ser Gln Arg
               20                  25                  30
   Thr Cys Ala Ala Phe Cys Arg Ser Leu Ser Cys Arg Lys Glu Gln Gly
           35                  40                  45
   Lys Phe Tyr Asp His Leu Leu Arg Asp Cys Ile Ser Cys Ala Ser Ile
       50                  55                  60
   Cys Gly Gln His Pro Lys Gln Cys Ala Tyr Phe Cys Glu Asn Lys Leu
   65                  70                  75                  80
   Arg Ser Gly Ser Gly Ser Gly Ser Gly Ser Arg Ser Met Ala Glu Thr
                   85                  90                  95
   Asp Pro Lys Thr Val Gln Asp Leu Thr Ser Val Val Gln Thr Leu Leu
               100                 105                 110
   Gln Glu Met Gln Asp Lys Phe Gln Thr Met Ser Asp Gln Ile Ile Glu
           115                 120                 125
   Arg Ile Asp Asp Met Ser Ser Arg Ile Asp Asp Leu Glu Lys Asn Ile
       130                 135                 140
   Ala Asp Leu Met Thr Gln Ala Gly Val Glu Glu Leu Glu Gly Ser Gly
   145                 150                 155                 160
   Ser Arg Ser Gly Gly His His His His His His
                   165                 170
<210> 19
   <211> 480
   <212> DNA
   <213> Artificial Sequence
<220>
   <223> NC-1 fragment
<221> CDS
   <222> (1)...(480)
<400> 19
   atg cct gag ggt ttt ata aag gca ggc caa agg ccc agt ctt tct ggg       48
   Met Pro Glu Gly Phe Ile Lys Ala Gly Gln Arg Pro Ser Leu Ser Gly
    1               5                  10                  15
   acc cct ctt gtt agt gcc aac cag cgg gta aca gga atg cct gtg tct       96
   Thr Pro Leu Val Ser Ala Asn Gln Arg Val Thr Gly Met Pro Val Ser
               20                  25                  30
   gct ttt act gtt att ctc tcc aaa gct tac cca gca ata gga act ccc      144
   Ala Phe Thr Val Ile Leu Ser Lys Ala Tyr Pro Ala Ile Gly Thr Pro
           35                  40                  45
   ata cca ttt gat aaa att ttg tat aac agg caa cag cat tat gac cca      192
   Ile Pro Phe Asp Lys Ile Leu Tyr Asn Arg Gln Gln His Tyr Asp Pro
       50                  55                  60
   agg act gga atc ttt act tgt cag ata cca gga ata tac tat ttt tca      240
   Arg Thr Gly Ile Phe Thr Cys Gln Ile Pro Gly Ile Tyr Tyr Phe Ser
   65                  70                  75                  80
   tac cac gtg cat gtg aaa ggg act cat gtt tgg gta ggc ctg tat aag      288
   Tyr His Val His Val Lys Gly Thr His Val Trp Val Gly Leu Tyr Lys
                   85                  90                  95
   aat ggc acc cct gta atg tac acc tat gat gaa tac acc aaa ggc tac      336
   Asn Gly Thr Pro Val Met Tyr Thr Tyr Asp Glu Tyr Thr Lys Gly Tyr
               100                 105                 110
   ctg gat cag gct tca ggg agt gcc atc atc gat ctc aca gaa aat gac      384
   Leu Asp Gln Ala Ser Gly Ser Ala Ile Ile Asp Leu Thr Glu Asn Asp
           115                 120                 125
   cag gtg tgg ctc cag ctt ccc aat gcc gag tca aat ggc cta tac tcc      432
   Gln Val Trp Leu Gln Leu Pro Asn Ala Glu Ser Asn Gly Leu Tyr Ser
       130                 135                 140
   tct gag tat gtc cac tcc tct ttc tca gga ttc cta gtg gct cca atg      480
   Ser Glu Tyr Val His Ser Ser Phe Ser Gly Phe Leu Val Ala Pro Met
   145                 150                 155                 160
<210> 20
   <211> 160
   <212> PRT
   <213> Artificial Sequence
<220>
   <223> NC-1 fragment
<400> 20
   Met Pro Glu Gly Phe Ile Lys Ala Gly Gln Arg Pro Ser Leu Ser Gly
    1               5                  10                  15
   Thr Pro Leu Val Ser Ala Asn Gln Arg Val Thr Gly Met Pro Val Ser
               20                  25                  30
   Ala Phe Thr Val Ile Leu Ser Lys Ala Tyr Pro Ala Ile Gly Thr Pro
           35                  40                  45
   Ile Pro Phe Asp Lys Ile Leu Tyr Asn Arg Gln Gln His Tyr Asp Pro
       50                  55                  60
   Arg Thr Gly Ile Phe Thr Cys Gln Ile Pro Gly Ile Tyr Tyr Phe Ser
   65                  70                  75                  80
   Tyr His Val His Val Lys Gly Thr His Val Trp Val Gly Leu Tyr Lys
                   85                  90                  95
   Asn Gly Thr Pro Val Met Tyr Thr Tyr Asp Glu Tyr Thr Lys Gly Tyr
               100                 105                 110
   Leu Asp Gln Ala Ser Gly Ser Ala Ile Ile Asp Leu Thr Glu Asn Asp
           115                 120                 125
   Gln Val Trp Leu Gln Leu Pro Asn Ala Glu Ser Asn Gly Leu Tyr Ser
       130                 135                 140
   Ser Glu Tyr Val His Ser Ser Phe Ser Gly Phe Leu Val Ala Pro Met
   145                 150                 155                 160
<210> 21
   <211> 195
   <212> DNA
   <213> Artificial Sequence
<220>
   <223> HSBP-1 fragment
<221> CDS
   <222> (1)...(195)
<400> 21
   atg gcc gaa act gat cct aaa aca gtt caa gac ctt acc agc gta gtc      48
   Met Ala Glu Thr Asp Pro Lys Thr Val Gln Asp Leu Thr Ser Val Val
    1               5                  10                  15
   cag acg ctc ctg caa gag atg caa gat aag ttt cag act atg agc gac      96
   Gln Thr Leu Leu Gln Glu Met Gln Asp Lys Phe Gln Thr Met Ser Asp
               20                  25                  30
   caa atc att gag aga atc gat gac atg agc tcc agg ata gat gac ctt     144
   Gln Ile Ile Glu Arg Ile Asp Asp Met Ser Ser Arg Ile Asp Asp Leu
           35                  40                  45
   gag aaa aat ata gca gat tta atg acg caa gct ggt gtg gaa gag ttg     192
   Glu Lys Asn Ile Ala Asp Leu Met Thr Gln Ala Gly Val Glu Glu Leu
       50                  55                  60
   gaa                                                                 195
<210> 22
   <211> 65
   <212> PRT
   <213> Artificial Sequence
<220>
   <223> HSBP-1 fragment
<400> 22
   Met Ala Glu Thr Asp Pro Lys Thr Val Gln Asp Leu Thr Ser Val Val
    1               5                  10                  15
   Gln Thr Leu Leu Gln Glu Met Gln Asp Lys Phe Gln Thr Met Ser Asp
               20                  25                  30
   Gln Ile Ile Glu Arg Ile Asp Asp Met Ser Ser Arg Ile Asp Asp Leu
           35                  40                  45
   Glu Lys Asn Ile Ala Asp Leu Met Thr Gln Ala Gly Val Glu Glu Leu
       50                  55                  60
   Glu
   65

## Description

The tumor necrosis factor (TNF) receptor superfamily is a large family of molecules involved in host defense, inflammation, and autoimmunity, and have been implicated in human disease. Therapeutic agents aimed at inhibiting TNF are effective in controlling inflammatory diseases such as rheumatoid arthritis and inflammatory bowel disease. Additional members of the TNF/TNF receptor superfamily are currently being targeted for therapies against autoimmune disease, atherosclerosis, osteoporosis allograft rejection and cancer.

Although both TNF and TNF receptor family members are active as self-assembling trimers, only functionally dimeric molecules, such as antibodies or receptor-IgG fusions, have been used as therapeutic agents. The three-fold symmetry displayed by both ligands and receptors in the TNF superfamily indicate that trimeric receptor based antagonists should display an increased avidity and therefore, increased effectiveness compared to dimeric molecules.

WO 98/39361 describes a lymphocyte surface receptor that binds CAML, nucleic acids encoding the same, and methods of use thereof.

Gross J. et al., Nature, Vol. 404, 27 April 2000, pages 995-999, reports that TACI and BCMA are receptors for a TNF homologue implicated in B-cell autoimmune disease.

Tai Li-Jung et al., J. Biol. Chem., Vol. 277, No.1, 4 January 2002, pages 735-745, describes that structure-function analysis of the heat-shock factor-binding protein reveals a protein composed solely of a highly conserved and dynamic coiled-coil trimerisation domain.

Frischholz S. et al., J. Biol. Chem., Vol. 273, No. 8, 20 February 1998, pages 4547-4555, describes characterisation of human type X procollagen and its NC-1 domain expressed as recombinant proteins in HEK293 cells.

Accordingly, a need still exists for a simple method for expressing TNF ligands and TNF receptors as stable trimers.

The present invention provides an isolated polypeptide, comprising (1) an extracellular domain of the transmembrane activator and CAML (calcium-signal modulating cyclophilin ligand) interactor (TACI), and (2) a trimerizing fragment of Heat Shock Binding Protein-1.

The present invention also provides use of a homotrimeric protein complex comprising a polypeptide, said polypeptide comprising (1) a TACI extracellular domain and (2) a trimerizing fragment of Heat Shock Binding Protein-1, for the manufacture of a medicament for inhibiting TNF4-induced B cell proliferation.

The present invention provides methods (as defined in the claims) for producing trimeric TNF receptors that are more potent inhibitors of their cognate ligand's biological activities, when compared to dimeric receptor molecules.

Suitable TACI extracellular domains include: (1) amino acid residues 30 to 110 of SEQ ID NO:4, (2) amino acid residues 1 to 110 of SEQ ID NO:4, (3) amino acid residues 30 to 154 of SEQ ID NO:4, and (4) amino acid residues 1 , to 154 of SEQ ID NO:4.

The present invention further provides homotrimeric complexes of fusion proteins comprising a TACI extracellular domain and a trimerizing fragment of Heat Shock Binding Protein-1, as defined in the claims.

These and other aspects of the invention will become evident upon reference to the following detailed description and drawing.

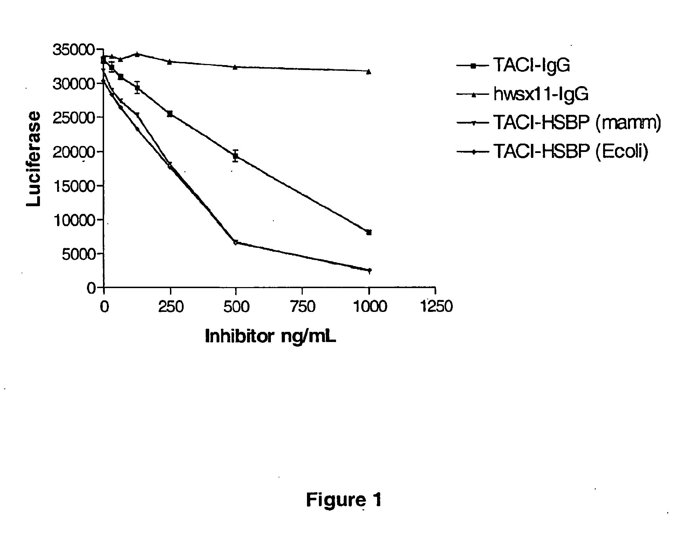

Figure 1 shows the inhibition of zTNF4-induced luciferase activity by a TACI-Fc fusion protein ("TACI-IgG"), a TACI-HSBP-1 protein produced in mammalian cells ("TACI-HSBP (mamm)"), a TACI-HSBP-1 protein produced in *E*. *coli* ("TACI-HSBP (Ecoli)"), and by a control immunoglobulin fusion protein ("hwsx11-IgG").

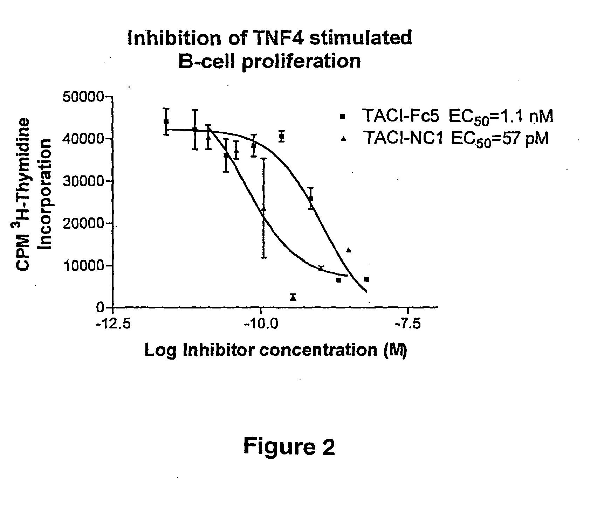

Figure 2 shows the inhibition of B-cell proliferation, through incorporation of ³H-thymidine, of the TACI-NCl trimer and TACI-Fc fusion protein ("TACI-Fc5"). TACI-Fc5 is an alternative name for TACI-Fc or TACI-IgG. Also noted on this figure are the EC₅₀ values (i.e., the concentration that inhibits the endpoint to 50% of the control) for the two molecules.

### 1. Definitions

In the description that follows, a number of terms are used extensively. The following definitions are provided to facilitate understanding of the invention.

As used herein, "nucleic acid" or "nucleic acid molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (*e*.*g*., α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

The term "complement of a nucleic acid molecule" refers to a nucleic acid molecule having a complementary nucleotide sequence and reverse orientation as compared to a reference nucleotide sequence. For example, the sequence 5' ATGCACGGG 3' is complementary to 5' CCCGTGCAT 3'.

The term "contig" denotes a nucleic acid molecule that has a contiguous stretch of identical or complementary sequence to another nucleic acid molecule. Contiguous sequences are said to "overlap" a given stretch of a nucleic acid molecule either in their entirety or along a partial stretch of the nucleic acid molecule. For example, representative contigs to the polynucleotide sequence 5' ATGGAGCTT 3' are 5' AGCTTgagt 3' and 3' tcgacTACC 5'.

The term "structural gene" refers to a nucleic acid molecule that is transcribed into messenger RNA (mRNA), which is then translated into a sequence of amino acids characteristic of a specific polypeptide. A "gene of interest" can be a structural gene.

"Complementary DNA (cDNA)" is a single-stranded DNA molecule that is formed from an mRNA template by the enzyme reverse transcriptase. Typically, a primer complementary to portions of mRNA is employed for the initiation of reverse transcription. Those skilled in the art also use the term "cDNA" to refer to a double-stranded DNA molecule consisting of such a single-stranded DNA molecule and its complementary DNA strand. The term "cDNA" also refers to a clone of a cDNA molecule synthesized from an RNA template.

An "isolated nucleic acid molecule" is a nucleic acid molecule that is not integrated in the genomic DNA of an organism. For example, a DNA molecule that encodes a growth factor that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

A "nucleic acid molecule construct" is a nucleic acid molecule, either single- or double-stranded, that has been modified through human intervention to contain segments of nucleic acid combined and juxtaposed in an arrangement not existing in nature.

"Linear DNA" denotes non-circular DNA molecules having free 5' and 3' ends. Linear DNA can be prepared from closed circular DNA molecules, such as plasmids, by enzymatic digestion or physical disruption.

A "promoter" is a nucleotide sequence that directs the transcription of a structural gene. Typically, a promoter is located in the 5' non-coding region of a gene, proximal to the transcriptional start site of a structural gene. Sequence elements within promoters that function in the initiation of transcription are often characterized by consensus nucleotide sequences. These promoter elements include RNA polymerase binding sites, TATA sequences, CAAT sequences, differentiation-specific elements (McGehee et al., Mol. Endocrinol. 7:551 (1993)), cyclic AMP response elements, serum response elements (Treisman, Seminars in Cancer Biol. 1:47 (1990)), glucocorticoid response elements, and binding sites for other transcription factors, such as CRE/ATF (O'Reilly et al., J. Biol. Chem. 267:19938 (1992)); AP2 (Ye et al., J. Biol. Chem. 269:25728 (1994)), SP1, cAMP response element binding protein (Loeken, Gene Expr. 3:253 (1993)) and octamer factors (see, in general, Watson et al., eds., Molecular Biology of the Gene, 4th ed. (The Benjamin/Cummings Publishing Company, Inc. 1987), and Lemaigre and Rousseau, Biochem. J. 303:1 (1994)). If a promoter is an inducible promoter, then the rate of transcription increases in response to an inducing agent. In contrast, the rate of transcription is not regulated by an inducing agent if the promoter is a constitutive promoter. Repressible promoters are also known.

A "core promoter" contains essential nucleotide sequences for promoter function, including the TATA box and start of transcription. By this definition, a core promoter may or may not have detectable activity in the absence of specific sequences that may enhance the activity or confer tissue specific activity.

A "regulatory element" is a nucleotide sequence that modulates the activity of a core promoter. For example, a regulatory element may contain a nucleotide sequence that binds with cellular factors enabling transcription exclusively or preferentially in particular cells, tissues, or organelles. These types of regulatory elements are normally associated with genes that are expressed in a "cell-specific," "tissue-specific," or "organelle-specific" manner.

An "enhancer" is a type of regulatory element that can increase the efficiency of transcription, regardless of the distance or orientation of the enhancer relative to the start site of transcription.

"Heterologous DNA" refers to a DNA molecule, or a population of DNA molecules, that does not exist naturally within a given host cell. DNA molecules heterologous to a particular host cell may contain DNA derived from the host cell species (*i.e*., endogenous DNA) so long as that host DNA is combined with non-host DNA. For example, a DNA molecule containing a non-host DNA segment that encodes a polypeptide operably linked to a host DNA segment comprising a transcription promoter is considered to be a heterologous DNA molecule. Conversely, a heterologous DNA molecule can comprise an endogenous gene operably linked with a promoter derived from a non-host gene. As another illustration, a DNA molecule comprising a gene derived from a wild-type cell is considered to be heterologous DNA if that DNA molecule is introduced into a mutant cell that lacks the wild-type gene.

A "polypeptide" is a polymer of amino acid residues joined by peptide bonds, whether produced naturally or synthetically. Polypeptides of less than about 10 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

A peptide or polypeptide synthesized within a cell from a heterologous nucleic acid molecule is a "heterologous" peptide or polypeptide.

An "integrated genetic element" is a segment of DNA that has been incorporated into a chromosome of a host cell after that element is introduced into the cell through human manipulation. Within the present invention, integrated genetic elements are most commonly derived from linearized plasmids that are introduced into the cells by electroporation or other techniques. Integrated genetic elements are passed from the original host cell to its progeny.

A "cloning vector" is a nucleic acid molecule, such as a plasmid, cosmid, or bacteriophage, that has the capability of replicating autonomously in a host cell. Cloning vectors typically contain one or a small number of restriction endonuclease recognition sites that allow insertion of a nucleic acid molecule in a determinable fashion without loss of an essential biological function of the vector, as well as nucleotide sequences encoding a marker gene that is suitable for use in the identification and selection of cells transformed with the cloning vector. Marker genes typically include genes that provide tetracycline resistance or ampicillin resistance.

An "expression vector" is a nucleic acid molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter.

A "recombinant host" is a cell that contains a heterologous nucleic acid molecule, such as a cloning vector or expression vector.

"Integrative transformants" are recombinant host cells, in which heterologous DNA has become integrated into the genomic DNA of the cells.

The term "expression" refers to the biosynthesis of a gene product. For example, in the case of a structural gene, expression involves transcription of the structural gene into mRNA and the translation of mRNA into one or more polypeptides.

The term "secretory signal sequence" denotes a DNA sequence that encodes a peptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

An "isolated polypeptide" is a polypeptide that is essentially free from contaminating cellular components, such as carbohydrate, lipid, or other proteinaceous impurities associated with the polypeptide in nature. Typically, a preparation of isolated polypeptide contains the polypeptide in a highly purified form, *i*.*e*., at least about 80% pure, at least about 90% pure, at least about 95% pure, greater than 95% pure, or greater than 99% pure. One way to show that a particular protein preparation contains an isolated polypeptide is by the appearance of a single band following sodium dodecyl sulfate (SDS)-polyacrylamide gel electrophoresis of the protein preparation and Coomassie Brilliant Blue staining of the gel. However, the term "isolated" does not exclude the presence of the same polypeptide in alternative physical forms, such as dimers or alternatively glycosylated or derivatized forms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

A "fusion protein" is a hybrid protein expressed by a nucleic acid molecule comprising nucleotide sequences of at least two genes. In this way, a fusion protein comprises as least two amino acid sequences that are not associated with each other in nature.

When used to describe a component of an expression vector, the language "gene or gene fragment" refers to a nucleotide sequence that encodes a polypeptide or peptide. The gene or gene fragment can be obtained from genomic DNA, from cDNA, or by an *in vitro* synthesis technique (*e*.*g*., polymerase chain reaction, chemical synthesis, and the like).

The term "affinity tag" is used herein to denote a polypeptide segment that can be attached to a second polypeptide to provide for purification or detection of the second polypeptide or provide sites for attachment of the second polypeptide to a substrate. In principal, any peptide or protein for which an antibody or other specific binding agent is available can be used as an affinity tag. Affinity tags include a polyhistidine tract, protein A (Nilsson et al., EMBO J. 4:1075 (1985); Nilsson et al., Methods Enzymol. 198:3 (1991)), glutathione S transferase (Smith and Johnson, Gene 67:31 (1988)), Glu-Glu affinity tag (Grussenmeyer et al., Proc. Natl. Acad. Sci. USA 82:7952 (1985)), substance P, FLAG peptide (Hopp et al., Biotechnology 6:1204 (1988)), streptavidin binding peptide, or other antigenic epitope or binding domain. See, in general, Ford et al., Protein Expression and Purification 2:95 (1991). DNA molecules encoding affinity tags are available from commercial suppliers (*e*.*g*., Pharmacia Biotech, Piscataway, NJ).

As used herein, the term "immunomodulator" includes cytokines, stem cell growth factors, lymphotoxins, co-stimulatory molecules, hematopoietic factors, and synthetic analogs of these molecules. Examples of immunomodulators include tumor necrosis factor, initerleukins, colony stimulating factors, interferons, stem cell growth factors, erythropoietin, and thrombopoietin.

The phrase an "immunoglobulin moiety" refers to a polypeptide that comprises a constant region of an immunoglobulin. For example, the immunoglobulin moiety can comprise a heavy chain constant region.

The phrase "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like.

An "antibody fragment" is a portion of an antibody such as F(ab')₂, F(ab)₂, Fab', Fab, and the like. Regardless of structure, an antibody fragment binds with the same antigen that is recognized by the intact antibody.

The term "antibody fragment" also includes a synthetic or a genetically engineered polypeptide that binds to a specific antigen, such as polypeptides consisting of the light chain variable region, "Fv" fragments consisting of the variable regions of the heavy and light chains, recombinant single chain polypeptide molecules in which light and heavy variable regions are connected by a peptide linker ("scFv proteins"), and minimal recognition units consisting of the amino acid residues that mimic the hypervariable region.

A "detectable label" is a molecule or atom which can be conjugated to a polypeptide to produce a molecule useful for identifying cells that express the binding partner of the polypeptide. Examples of detectable labels include chelators, photoactive agents, radioisotopes, fluorescent agents, paramagnetic ions, or other marker moieties.

Due to the imprecision of standard analytical methods, molecular weights and lengths of polymers are understood to be approximate values. When such a value is expressed as "about" X or "approximately" X, the stated value of X will be understood to be accurate to ±10%.

### 2. Expression Vectors For Producing Homotrimeric Polypeptides

The present invention provides methods as defined in the claims for producing homotrimeric proteins. Each protein of a homotrimer is a fusion protein that comprises a polypeptide of interest and a trimerizing fragment of HSBP-1. The Polypeptide of interest is an extracellular domain of TNFRSF13B (also known as "TACI"). Other useful tumor necrosis factor receptors are known to those of skill in the art.

The Examples illustrate the construction of fusion proteins comprising the extracellular domain of the transmembrane activator and CAML (calcium-signal modulating cyclophilin ligand) interactor (TACI). TACI nucleic acid and amino acid sequences are described by Bram and Gotz, U.S. Patent No. 5,969,102, and are included herein as SEQ ID NOs. 3 and 4. Illustrative TACI extracellular domains include polypeptides that have amino acid sequences comprising amino acid residues 30 to 110 of SEQ ID NO:4, amino acid residues 1 to 110 of SEQ ID NO:4, amino acid residues 30 to 154 of SEQ ID NO:4, and amino acid residues 1 to 154 of SEQ ID NO:4.

The Examples illustrate the use of amino acids 1 to 65 of the human heat shock factor binding protein, HSBP-1. HSBP-1 is described by Tai et al. J. Biol. Chem. 277:735 (2002). Nucleotide and amino acid sequences of a useful fragment of HSBP-1 are provided as SEQ ID NOs. 21 and 22.

In addition to the trimerizing amino acid sequences, the fusion protein can further comprise an immunoglobulin moiety in order to make the protein soluble. The immunoglobulin moiety can comprise a heavy chain constant region, such as a human heavy chain constant region. An IgG1 heavy chain constant region is one example of a suitable heavy chain constant region. An illustrative IgG1 heavy chain constant region is an IgG1 Fc fragment that comprises CH₂, and CH₃ domains. The IgG1 Fc fragment can be a wild-type IgG1 Fc fragment or a mutated IgG1 Fc fragment.

Expression vectors can be constructed that encode a fusion protein comprising a polypeptide of interest and a trimerizing amino acid sequence. Expression vectors that are suitable for production of a protein in eukaryotic cells typically contain (1) prokaryotic DNA elements coding for a bacterial replicationc origin and an antibiotic resistance marker to provide for the growth and selection of the expression vector in a bacterial host; (2) eukaryotic DNA elements that control initiation of transcription, such as a promoter; and (3) DNA elements that control the processing of transcripts, such as a transcription termination/polyadenylation signal sequence.

To express a gene, a nucleic acid molecule encoding the protein must be operably linked to regulatory sequences that control transcriptional expression and then, introduced into a host cell. In addition to transcriptional regulatory sequences, such as promoters and enhancers, expression vectors can include transcriptional and translational regulatory sequences. As an illustration, the transcriptional and translational regulatory signals suitable for a mammalian host may be derived from viral sources, such as adenovirus, bovine papilloma virus, simian virus, or the like, in which the regulatory signals are associated with a particular gene that has a high level of expression. Suitable transcriptional and translational regulatory sequences also can be obtained from mammalian genes, such as *actin, collagen, myosin,* and *metallothionein* genes.

Suitable transcriptional regulatory sequences include a promoter region sufficient to direct the initiation of RNA synthesis. Illustrative eukaryotic promoters include the promoter of the mouse *metallothionein 1* gene (Hamer et al., J. Molec. Appl. Genet. 1:273 (1982)), the *TK* promoter of *Herpes* virus (McKnight, Cell 31:355 (1982)), the *SV40* early promoter (Benoist et al., Nature 290:304 (1981)), the *Rous* sarcoma virus promoter (Gorman et al., Proc. Nat'l Acad Sci. USA 79:6777 (1982)), the cytomegalovirus promoter (Foecking et al., Gene 45:101 (1980)), and the mouse mammary tumor virus promoter (see, generally, Etcheverry, "Expression of Engineered Proteins in Mammalian Cell Culture," in Protein Engineering: Principles and Practice, Cleland et al. (eds.), pages 163-181 (John Wiley & Sons, Inc. 1996)).

Alternatively, a prokaryotic promoter, such as the *bacteriophage T3 RNA polymerase* promoter, can be used to control expression of the gene of interest in mammalian cells if the prokaryotic promoter is regulated by a eukaryotic promoter (Zhou et al., Mol. Cell. Biol. 10:4529 (1990), and Kaufman et al., Nucl. Acids Res. 19:4485 (1991)).

The inclusion of an affinity tag is useful for the identification or selection of cells displaying the fusion protein. Examples of affinity tags include polyHistidine tags (which have an affinity for nickel-chelating resin), *c-myc* tags (*e*.*g*., EQKLI SEEDL; SEQ ID NO:1) which are detected with anti-myc antibodies, calmodulin binding protein (isolated with calmodulin affinity chromatography), substance P, the RYIRS tag (which binds with anti-RYIRS antibodies), a hemagglutinin A epitope tag (*e*.*g*., YPYDV PDYA; SEQ ID NO:2) which is detected with an antibody, the Glu-Glu tag, and the FLAG tag (which binds with anti-FLAG antibodies). See, for example, Luo et al., Arch. Biochem. Biophys. 329:215 (1996), Morganti et al., Biotechnol. Appl. Biochem. 23:67 (1996), and Zheng et al., Gene 186:55 (1997). Nucleic acid molecules encoding such peptide tags are available, for example, from Sigma-Aldrich Corporation (St. Louis, MO).

The cloning site can be a multicloning site. Any multicloning site can be used, and many are commercially available. Particularly useful multicloning sites allow the cloning of a gene or gene fragment in all three reading frames.

The expression vector can include a nucleotide sequence that encodes a selectable marker. A wide variety of selectable marker genes are available (see, for example, Kaufman, Meth. Enzymol. 185:487 (1990); Kaufman, Meth. Enzymol. 185:537 (1990)). For example, one suitable selectable marker is a gene that provides resistance to the antibiotic neomycin. In this case, selection is carried out in the presence of a neomycin-type drug, such as G-418 or the like. *Bleomycin-resistance* genes, such as the *Sh ble* gene, are also useful selectable marker genes for the presently described methods. These genes produce a protein that inhibits the activity of bleomycin/phleomycin-type drugs, such as ZEOCIN (Gatignol et al., Mol. Gen. Genet. 207:342 (1987); Drocourt et al., Nucl. Acids Res. 18:4009 (1990)). ZEOCIN is toxic in a broad range of cell types, including bacteria, fungi, plant, avian, insect, and mammalian cells. Additional selectable markers include hygromycin B-phosphotransferase, the *AUR1* gene product, adenosine deaminase, aminoglycoside phosphotransferase, dihydrofolate reductase, thymidine kinase, and xanthine-guanine phosphoribosyltransferase (see, for example, Srivastava and Schlessinger, Gene 103:53 (1991); Romanos et al., "Expression of Cloned Genes in Yeast," in DNA Cloning 2: Expression Systems, 2nd Edition, pages 123-167 (IRL Press 1995); Markie, Methods Mol. Biol. 54:359 (1996); Pfeifer et al., Gene 188:183 (1997); Tucker and Burke, Gene 199:25 (1997); Hashida-Okado et al., FEBS Letters 425:117 (1998)). Selectable marker genes can be cloned or synthesized using published nucleotide sequences, or marker genes can be obtained commercially.

An expression vector can also include an *SV40* origin. This element can be used for episomal replication and rescue in cell lines expressing SV40 large T antigen.

A gene or gene fragment suitable for insertion into an expression vector can be obtained from cDNA, which is prepared by any method known in the art. For example, cDNA molecules can be synthesized by random priming. Moreover, such primers can be linked to restriction endonuclease sites found in the vector. Alternatively, cDNA molecules can be prepared by oligo d(T) priming. A gene or gene fragment can also be obtained from genomic DNA or by chemical synthesis. Standard methods for preparing suitable genes or gene fragments are known to those in the art (see, for example, Ausubel et al. (eds.), Short Protocols in Molecular Biology, 3rd Edition (John Wiley & Sons 1995) ["Ausubel 1995"]).

After constructing the expression vector, the vector can be propagated in a host cell to synthesize nucleic acid molecules for the generation of a nucleic acid polymer. Vectors, often referred to as "shuttle vectors," are capable of replicating in at least two unrelated expression systems. To facilitate such replication, the vector should include at least two origins of replication, one effective in each replication system. Typically, shuttle vectors are capable of replicating in a eukaryotic system and a prokaryotic system. This enables detection of protein expression in eukaryotic hosts, the "expression cell type," and the amplification of the vector in the prokaryotic hosts, the "amplification cell type." As an illustration, one origin of replication can be derived from *SV40,* while another origin of replication can be derived from *pBR322.* Those of skill in the art know of numerous suitable origins of replication.

Vector propagation is conveniently carried out in a prokaryotic host cell, such as *E. coli* or *Bacillus subtilus.* Suitable strains of *E. coli* include BL21(DE3), BL21(DE3)pLysS, BL21(DE3)pLysE, DH1, DH4I, DH5, DH5I, DH5IF', DH5IMCR, DH10B, DH10B/p3, DH11S, C600, HB101, JM101, JM105, JM109, JM110, K38, RR1, Y1088, Y1089, CSH18, ER1451, and ER1647 (see, for example, Brown (ed.), Molecular Biology Labfax (Academic Press 1991)). Suitable strains of *Bacillus subtilus* include BR151, YB886, MI119, MI120, and B170 (see, for example, Hardy, "Bacillus Cloning Methods," in DNA Cloning: A Practical Approach, Glover (ed.) (IRL Press 1985)). Standard techniques for propagating vectors in prokaryotic hosts are well-known to those of skill in the art (see, for example, Ausubel 1995; Wu et al., Methods in Gene Biotechnology (CRC Press, Inc. 1997)).

### 3. Production of Recombinant Protein by Host Cells

The expression vector can be introduced into any eukaryotic cell, such as a mammalian cell, insect cell, avian cell, fungal cell, and the like. Examples of suitable mammalian host cells include African green monkey kidney cells (Vero; ATCC CRL 1587), human embryonic kidney cells (293-HEK; ATCC CRL 1573), baby hamster kidney cells (BHK-21, BHK-570; ATCC CRL 8544, ATCC CRL 10314), canine kidney cells (MDCK; ATCC CCL 34), Chinese hamster ovary cells (CHO-K1; ATCC CCL61; CHO DG44 (Chasin et al., Som. Cell. Molec. Genet. 12:555, 1986)), rat pituitary cells (GH1; ATCC CCL82), HeLa S3 cells ((ATCC CCL2.2), rat hepatoma cells (H-4-II-E; ATCC CRL 1548) SV40-transformed monkey kidney cells (COS-1; ATCC CRL 1650) and murine embryonic cells (NIH-3T3; ATCC CRL 1658).

The baculovirus system provides an efficient means to introduce cloned genes of interest into insect cells. Suitable expression vectors are based upon the *Autographa californica* multiple nuclear polyhedrosis virus (AcMNPV), and contain well-known promoters such as *Drosophila heat shock protein (hsp)* 70 promoter, *Autographa californica nuclear polyhedrosis virus immediate-early* gene promoter (*ie-1*) and the *delayed early 39K* promoter, baculovirus *p10* promoter, and the *Drosophila metallothionein* promoter. A second method of making recombinant baculovirus utilizes a transposon-based system described by Luckow (Luckow, et al., J. Virol. 67:4566 (1993)). This system, which utilizes transfer vectors, is sold in the BAC-to-BAC kit (Life Technologies, Rockville, MD). This system utilizes a transfer vector, PFASTBAC (Life Technologies) containing a Tn7 transposon to move the gene or gene fragment into a baculovirus genome maintained in *E. coli* as a large plasmid called a "bacmid." See, Hill-Perkins and Possee, J. Gen. Virol. 71:971 (1990), Bonning, et al., J. Gen. Virol. 75:1551 (1994), and Chazenbalk, and Rapoport, J. Biol. Chem. 270:1543 (1995). These vectors can be modified following the above discussion

The recombinant virus or bacmid is used to transfect host cells. Suitable insect host cells include cell lines derived from IPLB-*Sf*-21, a *Spodoptera frugiperda.* pupal ovarian cell line, such as *Sf* 9 (ATCC CRL 1711), *Sf*21AE, and Sf21 (Invitrogen Corporation; San Diego, CA), as well as *Drosophila* Schneider-2 cells, and the HIGH FIVEO cell line (Invitrogen) derived from *Trichoplusia ni* (U.S. Patent No. 5,300,435). Commercially available serum-free media can be used to grow and to maintain the cells. Suitable media are Sf900 II™ (Life Technologies) or ESF 921™ (Expression Systems) for the Sf9 cells; and Ex-cellO405™ (JRH Biosciences, Lenexa, KS) or Express FiveO™ (Life Technologies) for the *T. ni* cells. When recombinant virus is used, the cells are typically grown up from an inoculation density of approximately 2-5 x 10⁵ cells to a density of 1-2 x 10⁶ cells at which time a recombinant viral stock is added at a multiplicity of infection (MOI) of 0.1 to 10, more typically near 3.

Established techniques for producing recombinant proteins in baculovirus systems are provided by Bailey et al., "Manipulation of Baculovirus Vectors," in Methods in Molecular Biology, Volume 7: Gene Transfer and Expression Protocols, Murray (ed.), pages 147-168 (The Humana Press, Inc. 1991), by Patel et al., "The baculovirus expression system," in DNA Cloning 2: Expression Systems, 2nd Edition, Glover et al. (eds.), pages 205-244 (Oxford University Press 1995), by Ausubel (1995) at pages 16-37 to 16-57, by Richardson (ed.), Baculovirus Expression Protocols (The Humana Press, Inc. 1995), and by Lucknow, "Insect Cell Expression Technology," in Protein Engineering: Principles and Practice, Cleland et al. (eds.), pages 183-218 (John Wiley & Sons, Inc. 1996).

The expression vectors described herein can also be used to transfect fungal cells, including yeast cells. Yeast species of particular interest in this regard include *Saccharomyces cerevisiae, Pichia pastoris,* and *Pichia methanolica.* Suitable promoters for expression in yeast include promoters from *GAL1* (galactose), *PGK* (phosphoglycerate kinase), *ADH* (alcohol dehydrogenase), *AOX1* (alcohol oxidase), HIS4 (histidinol dehydrogenase), and the like. Many yeast cloning vectors readily available and can be modified following the above discussion. These vectors include YIp-based vectors, such as YIp5, YRp vectors, such as YRp17, YEp vectors such as YEp13 and YCp vectors, such as YCp19. Methods for transforming S. *cerevisiae* cells with exogenous DNA and producing recombinant polypeptides therefrom are disclosed by, for example, Kawasaki, U.S. Patent No. 4,599,311, Kawasaki et al., U.S. Patent No. 4,931,373, Brake, U.S. Patent No. 4,870,008, Welch et al., U.S. Patent No. 5,037,743, and Murray et al., U.S. Patent No. 4,845,075. Transformed cells are selected by phenotype determined by the selectable marker, commonly drug resistance or the ability to grow in the absence of a particular nutrient (*e*.*g*., leucine). A preferred vector system for use in *Saccharomyces cerevisiae* is the *POT1* vector system disclosed by Kawasaki et al. (U.S. Patent No. 4,931,373), which allows transformed cells to be selected by growth in glucose-containing media. Additional suitable promoters and terminators for use in yeast include those from glycolytic enzyme genes (see, *e*.*g*., Kawasaki, U.S. Patent No. 4,599,311, Kingsman et al., U.S. Patent No. 4,615,974, and Bitter, U.S. Patent No. 4,977,092) and alcohol dehydrogenase genes. See also U.S. Patents Nos. 4,990,446, 5,063,154, 5,139,936, and 4,661,454.

Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson et al., J. Gen. Microbiol. 132:3459 (1986), and Cregg, U.S. Patent No. 4,882,279. *Aspergillus* cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming *Neurospora* are disclosed by Lambowitz, U.S. Patent No. 4,486,533.

For example, the use of *Pichia methanolica* as host for the production of recombinant proteins is disclosed by Raymond, U.S. Patent No. 5,716,808, Raymond, U.S. Patent No. 5,736,383, Raymond et al., Yeast 14:11-23 (1998), and in international publication Nos. WO 97/17450, WO 97/17451, WO 98/02536, and WO 98/02565. DNA molecules for use in transforming *P. methanolica* will commonly be prepared as double-stranded, circular plasmids, which are preferably linearized prior to transformation. For polypeptide production in *P. methanolica,* it is preferred that the promoter and terminator in the plasmid be that of a *P. methanolica* gene, such as a *P*. *methanolica* alcohol utilization gene (*AUG1* or *AUG2*). Other useful promoters include those of the dihydroxyacetone synthase (DHAS), formate dehydrogenase (FMD), and catalase (CAT) genes. To facilitate integration of the DNA into the host chromosome, it is preferred to have the entire expression segment of the plasmid flanked at both ends by host DNA sequences. For large-scale, industrial processes where it is desirable to minimize the use of methanol, it is preferred to use host cells in which both methanol utilization genes (*AUG1* and *AUG2*) are deleted. For production of secreted proteins, host cells deficient in vacuolar protease genes *(PEP4* and *PRB1)* are preferred. Electroporation is used to facilitate the introduction of a plasmid containing DNA encoding a polypeptide of interest into *P. methanolica* cells. *P. methanolica* cells can be transformed by electroporation using an exponentially decaying, pulsed electric field having a field strength of from 2.5 to 4.5 kV/cm, preferably about 3.75 kV/cm, and a time constant (t) of from 1 to 40 milliseconds, most preferably about 20 milliseconds.

An expression vector can be introduced into host cells using a variety of standard techniques including calcium phosphate transfection, liposome-mediated transfection, microprojectile-mediated delivery, electroporation, and the like.

Standard methods for introducing expression vectors into mammalian, yeast, and insect cells are provided, for example, by Ausubel (1995). General methods for expressing and recovering foreign protein produced by a mammalian cell system are provided by, for example, Etcheverry, "Expression of Engineered Proteins in Mammalian Cell Culture," in Protein Engineering: Principles and Practice, Cleland et al. (eds.), page 163 (Wiley-Liss, Inc. 1996). Established methods for isolating recombinant proteins from a baculovirus system are described by Richardson (ed.), Baculovirus Expression Protocols (The Humana Press, Inc. 1995).

Expression vectors can be isolated from cells that produce a polypeptide of interest. If desired, expression vectors can be subjected to another round of selection based on expression of the identifiable polypeptide or, transfected into the amplification cell type. The transfected amplification cell type is then selected by the selectable marker, the vectors are purified and the nucleotide sequence of the gene or gene fragment is sequenced by any method known in the art. If the nucleotide sequence encodes only a portion of a complete polypeptide, then the nucleotide sequence can be used as a probe by methods known in the art to retrieve the entire gene.

The present invention, thus generally described, will be understood more readily by reference to the following examples, which are provided by way of illustration and are not intended to be limiting of the present invention.

### COMPARATIVE EXAMPLE 1

### Construction of TACI-NC1

An expression vector was constructed that encodes a fusion comprising an extracellular domain of the transmembrane activator and CAML (calcium-signal modulating cyclophilin ligand) interactor (TACI) protein and the NC1 domain of human collagen X. TACI nucleic acid sequences are described by Bram and Gotz, U.S. Patent No. 5,969,102, and included herein as SEQ ID NOs. 3 and 4. The NC-1 domain is described by Frischholz et al., J. Biol. Chem. 273:4547 (1998); nucleotide and amino acid sequences are provided herein has SEQ ID NOs. 19 and 20. In this construct, the extracellular domain of TACI was fused to NC1 with a Glu-Glu tag at the c-terminus and a Gly-Ser spacer of eight amino acids engineered between TACI and NC1.

NC1 was amplified by PCR from human genomic DNA (Clontech) using oligonucleotides zc40219 (5' GGGCCTCCAG GCCCACCAGG T 3'; SEQ ID NO:5) and zc40205 (5' TCACATTGGA GCCACTAGGA A 3'; SEQ ID NO:6). The extracellular portion of TACI was amplified by PCR from a clone that encoded a TACI-immunoglobulin fusion protein with oligonucleotides zc40915 (5' ACAGGTGTCC AGGGAATTCA TATAGGCCGG CCACCATGGA TGCAATGAAG AGAGGG 3'; SEQ ID NO:7) and zc40917 (5' ACCCTCAGGC ATCGAACCCG AACCCGAACC GGATCC 3': SEQ ID NO:8) with conditions of 30 cycles of 94°C for one minute, 55°C for one minute, and 72°C for two minutes. The PCR products were precipitated and resuspended in 10 µl of water and then recombined in S *cerevisiae* into pZMP21 that had been digested with *Bgl*II*. E. coli* clones that resulted from the recombination were screened for proper incorporation by AscI digestion and three positive clones were submitted for sequencing. One clone was selected for further study. This clone contained a glycine to arginine mutation in NC1 and lacked four amino acids from the C-terminal Glu-Glu tag.

A vector encoding TACI/NC1-EE was linearized for electroporation by digesting 20 µg of Qiagen-purified DNA with *Pvu*I. This linearized DNA was electroporated in PF-CHO cells. The cells were allowed to recover for 24-hours before nutrient selection in HT- media for ten days. After recovery from nutrient selection, cells were transferred into 50 nm methotrexate selection for an additional ten days.

Transfected cells were seeded into cell factories and two liters of factory conditioned media (CM) was isolated. The CM was combined with 1.5 mg Anti-TACI monoclonal antibody and incubated overnight at 4°C. The CM-antibody mixture was applied to a 1.6 mL bed volume POROS A50 column at a flow rate of 2 mL/min. Following addition, the column was washed with 100 column volumes of PBS, pH 7.2. The bound protein was then eluted directly into 2 M tris pH 8.0 with 200 mM glycine pH2.5. One milliliter fractions were collected. Based on western blot analysis, fractions containing TACI-NC1 were pooled. The pooled fractions were concentrated to 300 µl, and buffer exchanged three times with 14 mL PBS, pH 7.2, and then dialyzed against three changes of 4 L PBS, pH 7.2.

### EXAMPLE 2

### Expression of TACI-HSBI in Mammalian Cells

### A. Synthesis of the HSB1 gene

Human heat shock binding protein (HSBP-1) is described by Tai et al. J. Biol. Chem. 277:735 (2002). HSBP-1 nucleotide and amino acid sequences are provided as SEQ ID NOs. 21 and 22. Four overlapping oligonucleotides, which encoded both sense and antisense strands of human heat shock binding protein (HSBP-1), were synthesized by solid phased synthesis, using the following primers: 5' GATCGGATCC ATGGCCGAAA CTGATCCTAA AACAGTTCAA GACCTTACCA GCGTAGTCCA GACGCTCCTG CAAGAGATCG AAGATAAGTT TCAGACTATG AGCGACCAAA TCATTGAG 3' (SEQ ID NO:9); 5' AGAATGCATG ACATGAGCTC CAGGATAGAT GACCTTGAGA AAAATATAGC AGATTTAATG ACGCAAGCTG GTGTGGAAGA GTTGGAAGGA AGTGGTTCTA 3' (SEQ ID NO:10); 5' GATCTAGAAC CACTTCCTTC CAACTCTTCC ACACCAGCTT GCGTCATTAA ATCTGCTATA TTTTTCTCAA GGTCATCTAT CCTGGAGCTC ATGTCATCGA TTCTCTCAAT 3' (SEQ ID NO:11); and 5' GATTTGGTCG CTCATAGTCT GAAACTTATC TTGCATCTCT TGCAGGAGCG TCTGGACTAC GCTGGTAAGG TCTTGAACTG TTTTAGGATC AGTTTCGGCC ATGGATCC 3' (SEQ ID NO: 12). The 5' end of each oligonucleotide was phosphorylated by combining 120 pmoles of each oligonucleotide, 1.6 µl 100 mM ATP, 34 µl 5x T4-Kinase buffer (Life Technologies, Bethesda, MD), 6.4 µl water, and 1 µl T4-polynucleotide kinase (Life Technologies), and incubating for 20 minutes at 37°C. The phosphorylation reaction was placed into a boiling water bath and then slowly cooled to 25°C to promote annealing. The fragments were ligated by adding 20 µl of 10x T4-ligase buffer (Life Technologies), 0.5 µl of 100 mM ATP, and 2 µl of T4-DNA ligase (Life Technologies), and incubating overnight at 16°C. Following ligation, the DNA was collected by alcohol precipitation. The isolated DNA was resuspended in 3.2 µl of B restriction buffer (Promega, Madison, WI), 26.8 µl of water, 1.5 µl of *Bgl*II (Life Technologies), and 1.5 µl of Asp718 (Life Technologies), and incubated for two hours at 37°C. The ligase reaction product was fractionated on a 15% agarose gel and the 220 nucleotide fragment encoding human HSBP-1 was isolated using a Qiagen gel isolation kit according to manufacturer's protocol (Qiagen). The human HSBP-1 was inserted into an *Asp*718-BamHI cleaved vector using T4-DNA ligase and manufacturer's guidelines (Life Technologies).

A fragment encoding the extracellular domain of TACI was amplified from the pTACI-NC1 vector using the oligonucleotides zc41712 (5' CACACGTACG AAGATGGATG CAATGAAGAG AGG 3'; SEQ ID NO:13) and zc41638 (5' GGTTAGATCT CGAACCCGAA CCCGAACCGG 3'; SEQ ID NO:14). The PCR product was cut with *Bsi*W1 and *Bg*/II*,* and the amplified DNA fractionated on 1.5% agarose gel and then isolated using a Qiagen gel isolation kit according to manufacturer's protocol (Qiagen). The isolated DNA was inserted into *Asp*718-*Bg*III cleaved vector that included the HSBP-1-enconding sequence, using T4-DNA ligase, following the manufacturer's guidelines (Life Technologies). DNA sequencing confirmed the expected sequence of the vector, which was designated "pHZTACI-HSBP.9."

### B. Expression and Purification of TACI-HSBP-1

The pHZTACI-HSBP.9 vector was transfected into BHK570 using TransTransfected and the cultures were selected for trasnfectants resistance to 10 µM methotrexate. Resistant colonies were transferred to tissue culture dishes, expanded and analyzed for secretion of TACI-HSBP-His₆ by western blot analysis with Anti-His (C-terminal) Antibody (Invitrogen, Carlsbad, CA). The resulting cell line, "BHK.TACI-HSBP.2," was expanded.

BHK.TACI-HSBP.2 cells were seeded into cell factories and 12 liters of factory-conditioned media were isolated. The media were applied to a 25 milliliter bed volume Ni-NTA (Invitrogen) at a flow rate of two ml/min. Following addition, the column was washed with 50 column volumes of PBS, pH 7.2 and 20 column volumes of phosphate buffered saline, 50 mM imidizole, pH 6.0. The bound protein was then eluted with a linear gradient of increasing imidizole concencentration from 50 mM to 800 mM in PBS, pH 6.0, at 1 ml/min for 60 minutes. One milliliter fractions were collected. Based on western blot analysis, fractions containing TACI-HSBP-1 were pooled. The pooled fractions were concentrated to 300 µl, and buffer was exchanged three times with 14 ml of phosphate buffered saline (pH 7.2), and then dialyzed against three changes of four liters of phosphate buffered saline, pH 7.2. Western blot analysis and coomasie stained gels showed greater than 75% purity and a monomeric molecular weight of 20 kDa.

### EXAMPLE 3

### Proliferation Assay for TACI-fusion Proteins

Peripheral blood mononuclear cells from apheresis were isolated by density gradient centrifugation on Ficol-Hypaque and washed in phosphate buffered saline. Typically, about 10¹⁰ peripheral blood mononuclear cells can be isolated from one donor. About 10⁸ cells were frozen per vial in 90% fetal calf serum and 10% dimethylsulfoxide.

Multiple vials were thawed and cell viability was determined. B cells were isolated from peripheral blood mononuclear cells using CD19 magnetic beads and the VarioMacs magnetic separation system (Miltenyi Biotec; Auburn, CA). Round bottom 96 well plates were pre-coated with goat anti-human IgM at 5 µg/ml in phosphate buffered saline for 24 hours at 4°C. (Southern Biotechnology Assoc. Inc.; Birmingham AL). Purified B cells were plated at 10⁵ cells per well in the presence of 10 ng/mL human IL-4 (Pharmingen) and 20 ng/mL zTNF4, a ligand that binds with TACI. A three-fold dilution series of TACI-Fc fusion protein, TACI-HSBP-1, or TACI-NC1 starting at 1 µg/ml, were included to compare their ability to inhibit zTNF4 stimulated B-cell proliferation. The cells were incubated for four days in the presence of zTNF4, human IL-4 and with or without inhibitors, and then pulsed overnight with 1 µCi of H³ thymidine (Amersham) per well. Plates were harvested using a Packard plate harvester and counted using the Packard reader. TACI-HSBP-1 was found to be three-fold more efficient, and TACI-NC1 ten-fold more efficient at inhibiting zTNF4 stimulated proliferation of human B-cells in this assay. As shown in Figure 2, TACI-NC1 reduced the TNF4 induced B-cell proliferation to a greater degree than the same concentration of TACI-Fc5. This difference is numerically expressed in the EC₅₀ values indicated in the figure, with TACI-Fc5 having a value of 1.1 nM and TACI-NC1 having a value of 57 µM, indicating that TACI-NC1 is approximately 19 times more effective.

### EXAMPLE 4

### Expression of TACI-HSB1 in Bacterial Cells

An expression plasmid containing a nucleotide sequence that encodes human TACI-HSBP-1, was inserted behind the G10 enhancer sequence via yeast homologous recombination. A DNA fragment of human TACI-HSBP-1 was isolated using PCR. Two primers were used in the production of human TACI-HSBP-1 in a PCR reaction. Primer zc42,728 (5' CTAGAAATAA TTTTGTTTAA CTTTAAGAAG GAGATATATA TATGGCTATG AGATCCTGCC CC 3'; SEQ ID NO:15) containing 40 base pairs of vector flanking sequence, comprised of the g10 enhancer sequence and 24 base pairs corresponding to the amino terminus of human TACI-HSBP-1. Primer zc42,731 (5' TCTGTATCAG GCTGAAAATC TTATCTCATC CGCCAAAACA CTAGTGATGG TGATGGTGAT GGCC 3'; SEQ ID NO: 16) contained 40 base pairs of the vector flanking sequence and 24 base pairs corresponding to the carboxyl terminus of the TACI-HSBP-1 sequence. The template was pH2-TACI-HSBP9. The PCR reaction conditions were as follows: 25 cycles of 94°C for 30 seconds, 50°C for 30 seconds, and 72°C for 1 minute; followed by a 4°C soak. A 2 to 4 µl volume of the PCR sample was run on a 1% agarose gel with 1xTBE buffer for analysis, and the expected band of approximately 550 base pair fragment (5' ATGGCTATGA GATCCTGCCC CGAAGAGCAG TACTGGGATC CTCTGCTGGG TACCTGCATG TCCTGCAAAA CCATITGCAA CCATCAGAGC CAGCGCACCT GTGCAGCCTT CTGCAGGTCA CTCAGCTGCC GCAAGGAGCA AGGCAAGTTC TATGACCATC TCCTGAGGGA CTGCATCAGC TGTGCCTCCA TCTGTGGACA GCACCCTAAG CAATGTGCAT ACTTCTGTGA GAACAAGCTC AGGAGCGGAT CCGGTTCGGG TTCGGGTTCG AGATCCATGG CCGAAACTGA TCCTAAAACA GTTCAAGACC TTACCAGCGT AGTCCAGACG CTCCTGCAAG AGATGCAAGA TAAGTTTCAG ACTATGAGCG ACCAAATCAT TGAGAGAATC GATGACATGA GCTCCAGGAT AGATGACCTT GAGAAAAATA TAGCAGATTT AATGACGCAA GCTGGTGTGG AAGAGTTGGA AGGAAGTGGT TCTAGATCCG GTGGCCATCA CCATCACCAT CACTGA 3'; SEQ ID NO: 17) (MAMRSCPEEQ YWDPLLGTCM SCKTICNHQS QRTCAAFCRS LSCRKEQGKF YDHLLRDCIS CASICGQHPK QCAYFCENKL RSGSGSGSGS RSMAETDPKT VQDLTSWQT LLQEMQDKFQ TMSDQIIERI DDMSSRII7DL EKNIADLMTQ AGVEELEGSG SRSGGHHHHH H; SEQ ID NO: 18) was observed. The remaining volume of the 100 µl reaction was precipitated with 200 µl absolute ethanol. The pellet was resuspended in 10 µl water to be used for recombining into Smal-cleaved recipient vector pTAP238 to produce the construct encoding TACI-HSBP-1.

One hundred microliters of competent yeast cells (*S*. *cerevisiae)* were combined with 10 µl of a mixture containing approximately 1 µg of each of the human TACI-HSBP-1 fragments (PCR products) and 100 ng of *Sma*I-digested pTAP238 vector, and transferred to a 0.2-cm electroporation cuvette. The yeast/DNA mixture was electropulsed using instrument settings of 0.75 kV (5 kV/cm), infinite ohms, 25 µF, and then 600 µl of 1.2 M sorbitol were added to the cuvette. The yeast was then plated in two 300-µl aliquots onto two -URA D (glucose-containing media lacking uracil) plates and incubated at 30°C. After about 48 hours, the Ura+ yeast transformants from a single plate were resuspended in 1 ml of water and spun briefly to pellet the cells. The cell pellet was resuspended in 1 ml of lysis buffer. DNA was recovered as disclosed above. The DNA pellet was resuspended in 100 µl of water.

Forty µl of electrocompetent *E. coli* MC1061 cells were transformed with 1 µl of the yeast DNA. The cells were electropulsed at 2.0 kV, 25 µF and 400 ohms. Following electroporation, 0.6 ml SOC (2% Bacto^{™} Tryptone (Difco, Detroit, MI), 0.5% yeast extract (Difco), 10 mM NaCl, 2.5 mM KCI, 10 mM MgCl₂, 10 mM MgSO₄, 20 mM glucose) was added to the cells. The cells were allowed to recover at 37°C for one hour, then were plated in one aliquot on LB + kanamycin plates (LB broth (Lennox), 1.8% Bacto^{™} Agar (Difco), 30 mg/L kanamycin).

Individual clones harboring the correct expression construct for human TACI-HSBP-1 were identified by diagnostic digest of the plasmid DNA. Cells were grown in Super Broth II (Becton Dickinson) with 30 µg/ml of kanamycin overnight. The next day, the cells were harvested, and plasmid DNA was prepared using spin columns (QlAprep® Spin Miniprep Kit; Qiagen Inc., Valencia, CA). The DNA was then cleaved with *Not*I and *Xba*I. The clones with the correct restriction pattern were designated pTAP415 and sequenced. The polynucleotide sequence of TACI-HSBP-1 in pTAP415 is shown in SEQ ID NO: 17.

Ten microliters of pTAP415 were cleaved with two microliters *of Not*I in 3 µl of a commercially available buffer (buffer 3; New England Biolabs) and 15 µl of water for one hour at 37°C. Seven microliters of the reaction mixture were combined with two microliters of 5x T4 DNA ligase buffer (Life Technologies; Gaithersburg, MD) and one microliter of T4 DNA ligase and incubated at room termperature for one hour. One microliter of the ligation mixture was used to transform *E*. *coli* strain W3110 (ATCC 27325). The cells were electropulsed at 2.0 kV, 25 µF, and 400 ohms. Following electroporation, 0.6 ml of SOC was added to the cells. The cells were grown at 37°C for one hour, then plated in one aliquot on LB + kanamycin plates.

Individual colonies were picked and grown. Plasmid DNA was prepared using spin columns. The DNA was cut diagnostically with *Pvu*II and *Hind*III to confirm the loss of yeast URA3 and CEN/ARS elements. An individual colony was picked. Cells were grown in Superbroth II (Becton Dickinson) containing 30 µg/ml of kanamycin overnight. One hundred microliters of the overnight culture were used to inoculate two milliliters of fresh Superbroth II containing 30 µg/ml kanamycin. Cultures were grown at 37°C with shaking for about 2 hours in 15-ml conical tubes. One ml of the culture was induced with 1 mM IPTG. Two hours and 15 minutes later, an equal volume of culture was mixed with 250 µl of Thorner buffer (8M urea, 100mM Tris pH 7.0, 10% glycerol, 2mM EDTA, 5% SDS) with 5% βME and dye. Samples were boiled for five minutes. Twenty-µl samples were loaded on a 4%-12% PAGE gel (NOVEX). Gels were run in 1 x MES buffer. Expression was analyzed by Coomassie Blue staining.

Bacterial cells were lysed using a French press, and inclusion bodies in the cell lysate were pelleted by low-speed centrifugation. The pellet fraction was washed with 2M urea to remove contaminants including membrane and cell wall material. TACI-HSBP-1 *E. coli* inclusion bodies were then extracted overnight with stirring at 4°C in 7 M guanidine HCl in 50 mM Tris pH 8 containing 0.1 M sodium sulfite and 0.05 M sodium tetrathionate. Extraction with the denaturant/sulfitolysis reagents simultaneously dissociates protein-protein interactions and unfolds the protein to monomer with sulfhydryl groups in the reduced state and sulphonated state. Before refolding, samples were centrifuged for 30 minutes at 35,000xg at 4°C and filtered with a 0.2 µm filter. Concentrations were estimated by a RP HPLC assay.

### EXAMPLE 5

### Biological Assay for TACI-fusion Proteins

A Jurkat cell line transfected with human TACI and with KZ142 Luciferase was used to test the ability of various trimerizing TACI constructs to neutralize zTNF4 acitvity. The Jurkat TACI KZ142 Luciferase cells were grown and assayed in growth media (RPMI/10%FBS with L-glutamine, sodium pyruvate, 0.5 mg/ml G418, and 2 µg/ml puromycin). These cells were plated at 40,000 cells/96-well in 100 µl of growth media. One hundred microliters per well of trimerizing inhibitor weres added in the presence of 100 ng/ml of zTNF4.

The assay cell line showed a maximal approximate 18-fold luciferase responsiveness to 1000 ng/ml of zTNF4. One hundred nanograms per milliliter of zTNF4 gave an approximate 10-fold luciferase responsiveness compared to the background control well. The combination of zTNF4 and inhibitor in a total of 200 µl of growth media was incubated for six hours at 37°C in a 5% CO₂ incubator. The 96-well plate was then centrifuged at 2000xg in a Beckman GS-6KR centrifuge and media were discarded by a quick inversion of the plate. Twenty five microliters of lysis buffer (Promega E153A) were added to each well and incubated for 15 minutes at room temperature. The lysed cells were then transferred to an opaque 96-well plate for purposes of luminometer readings. One bottle of luciferase assay buffer (Promega 152A) was added to one bottle of luciferase assay substrate (Promega E151A) and 40 µl of this combination were added to each well. Each well was read on a luminometer (EG&G Berthold Microlumat Plus) with five seconds of integration.

As shown in Figure 1, a control immunoglobulin fusion protein (hwsx11-IgG) had little effect on the zTNF4-induced luciferase activity. Although a TACI-Fc immunoglobulin fusion protein (TACI-IgG) inhibited luciferase activity, greater inhibition was achieved with TACI-HSBP-1 proteins produced in mammalian cells or in *E*. *coli.*

## Claims

1. An isolated polypeptide, comprising (1) an extracellular domain of the transmembrane activator and CAML (calcium-signal modulating cyclophilin ligand) interactor (TACI), and (2) a trimerizing fragment of Heat Shock Binding Protein-1.

2. A homotrimeric protein complex, comprising the polypeptide of Claim 1.

3. The isolated polypeptide of Claim 1, wherein the TACI extracellular domain is selected from the group consisting of: (1) amino acid residues 30 to 110 of SEQ ID NO:4, (2) amino acid residues 1 to 110 of SEQ ID NO:4, (3) amino acid residues 30 to 154 of SEQ ID NO:4, and (4) amino acid residues 1 to 154 of SEQ ID NO:4.,

4. The isolated polypeptide of Claim 1, wherein the trimerizing fragment of Heat Shock Binding Protein-1 comprises the amino acid sequence of SEQ ID NO:22.

5. The isolated polypeptide of Claim 4, wherein the TACI extracellular domain comprises the amino acid residues 30 to 110 of SEQ ID NO: 4.

6. A homotrimeric protein complex, comprising the polypeptide of Claim 5.

7. An expression vector comprising the following operably linked elements:
a transcription promoter;
the nucleic acid sequence encoding the polypeptide of Claim 1 and
a transcription terminator.

8. A cultured cell into which has been introduced the expression vector of Claim 7, wherein said cell expresses said polypeptide.

9. A method of producing a homotrimeric protein complex comprising the steps of culturing the cell of Claim 8 and recovering the homotrimeric protein complex comprising said polypeptide.

10. Use of a homotrimeric protein complex comprising a polypeptide, said polypeptide comprising (1) a TACI extracellular domain and (2) a trimerizing fragment of Heat Shock Binding Protein-1, for the manufacture of a medicament for inhibiting TNF4-induced B cell proliferation.

11. Use of Claim 10 wherein said homotrimeric complex is the complex of Claim 6.

12. An isolated polypeptide according to Claim 1, wherein said polypeptide comprises an affinity tag selected from the group consisting of a polyhistidine tag, calmodulin binding protein tag, substance P tag, the RYIRS tag, hemagglutinin A epitope tag, Glu-Glu tag and the FLAG tag.

13. A homotrimeric protein complex comprising a polypeptide, said polypeptide comprising (1) a TACI extracellular domain and (2) a trimerizing fragment of Heat Shock Binding Protein-1, for inhibiting TNF4-induced B cell proliferation.

## Patentansprüche

1. Ein isoliertes Polypeptid, umfassend (1) einen extrazellulären Bereich des transmembranen Aktivators und CAML (Calcium-Signal modulierendes Cyclophilin Ligand) Wechselwirker (TACI), und (2) ein trimerisierendes Fragment des Hitzeschock-Bindungsproteins-1.

2. Ein homotrimerischer Proteinkomplex, umfassend das Polypeptid nach Anspruch 1.

3. Das isolierte Polypeptid nach Anspruch 1, wobei der TACI extrazelluläre Bereich aus der Gruppe ausgewählt ist bestehend aus: (1) Aminosäurerückstände 30 - 110 der SEQ ID Nr. 4, (2) Aminosäurerückstände 1 - 110 der SEQ ID Nr. 4, (3) Aminosäurerückstände 30 - 154 der SEQ ID Nr. 4, und (4) Aminosäurerückstände 1 - 154 der SEQ ID Nr. 4.

4. Das isolierte Polypeptid nach Anspruch 1, wobei das trimerisierende Fragment des Hitzeschock-Bindungsproteins-1 die Aminosäuresequenz der SEQ ID Nr. 22 umfasst.

5. Das isolierte Polypeptid nach Anspruch 4, wobei der TACl extrazelluläre Bereich die Aminosäurerückstände 30 - 110 der SEQ ID Nr. 4 umfasst.

6. Ein homotrimerischer Proteinkomplex, umfassend das Polypeptid nach Anspruch 5.

7. Ein Expressionsvektor umfassend die folgenden operabel verbundenen Elemente:
ein Transkriptionspromotor;
die Nucleinsäuresequenz, die das Polypeptid nach Anspruch 1 verschlüsselt und einen Transkriptionsterminator.

8. Eine kultivierte Zelle, in welche der Expressionsvektor nach Anspruch 7 eingebracht wurde, wobei diese Zelle das Polypeptid exprimiert.

9. Ein Verfahren zur Herstellung eines homotrimerischen Proteinkomplexes, umfassend die Schritte des Kultivierens der Zelle nach Anspruch 8 und des Aufbereitens des homotrimerischen Proteinkomplexes umfassend das Polypeptid.

10. Verwendung von einem homotrimerischen Proteinkomplex umfassend ein Polypeptid, das Polypeptid umfassend (1) einen TACl extrazellulären Bereich und (2) ein trimerisierendes Fragment des Hitzeschock-Bindungsproteins-1, zur Erzeugung eines Medikaments zum Verhindern der Proliferation von TNF4-induzierten B-Zellen.

11. Verwendung nach Anspruch 1 wobei der homotrimerische Komplex der Komplex nach Anspruch 6 ist.

12. Ein isoliertes Polypeptid gemäß Anspruch 1, wobei das Polypeptid eine Affinitäts-Markierung umfasst ausgewählt aus der Gruppe bestehend aus einer Polyhistidin-Markierung, Calmodulinbindungsprotein-Markierung, Substanz-P-Markierung, der RYIRS-Markierung, Hemagglutinin-A-Epitop-Markierung, Glu-Glu-Markierung und der FLAG-Markierung.

13. Ein homotrimerischer Proteinkomplex umfassend ein Polypeptid, das Polypeptid umfassend (1) einen TACl extrazellulären Bereich und (2) ein trimerisierendes Fragment des Hitzeschock-Bindungsproteins-1, zum Verhindern der Proliferation von TNF4-induzierten B-Zellen.

## Revendications

1. Polypeptide isolé, comprenant (1) un domaine extracellulaire de l'activateur transmembranaire, et un interacteur de CAML (ligand de cyclophiline modulant le signal de calcium) (TACI) et (2) un fragment de trimérisation de la protéine de liaison de choc thermique-1.

2. Complexe protéique homotrimérique, comprenant le polypeptide selon la revendication 1.

3. Polypeptide isolé selon la revendication 1, dans lequel le domaine extracellulaire TACI est choisi dans le groupe constitué de : (1) résidus d'acide aminé 30 à 110 de SEQ. ID N°4, (2) résidus d'acides aminés 1 à 110 de SEQ. ID N°4, (3) résidus d'acides aminés 30 à 154 de SEQ. ID N°4, et (4) résidus d'acides aminés 1 à 154 de SEQ. ID N°4.

4. Polypeptide isolé selon la revendication 1, dans lequel le fragment de trimérisation de la protéine de liaison de choc thermique-1 comprend la séquence d'acide aminé de SEQ. ID N°22.

5. Polypeptide isolé selon la revendication 4, dans lequel le domaine extracellulaire TACI comprend les résidus d'acides aminés 30 à 110 de SEQ. ID N°4.

6. Complexe protéique homotrimérique, comprenant le polypeptide selon la revendication 5.

7. Vecteur d'expression comprenant les éléments opérationnellement liés suivants :
un promoteur de transcription ;
la séquence d'acide nucléique codant le polypeptide de la revendication 1 et un terminateur de transcription.

8. Cellule cultivée dans laquelle a été introduit le vecteur d'expression selon la revendication 7, dans laquelle ladite cellule exprime ledit polypeptide.

9. Procédé de production d'un complexe protéique homotrimérique comprenant les étapes consistant à cultiver la cellule de la revendication 8, et à récupérer le complexe protéique homotrimérique comprenant ledit polypeptide.

10. Utilisation d'un complexe protéique homotrimérique comprenant un polypeptide, ledit polypeptide comprenant (1) un domaine extracellulaire TACI et (2) un fragment de trimérisation de la protéine de liaison de choc thermique-1 pour la fabrication d'un médicament destiné à inhiber la prolifération de lymphocytes B induite par la TNF4.

11. Utilisation selon la revendication 10, dans laquelle ledit complexe homotrimérique est le complexe selon la revendication 6.

12. Polypeptide isolé selon la revendication 1, dans lequel ledit polypeptide comprend un marqueur d'affinité choisi dans le groupe constitué de marqueur polyhistidine, de marqueur par protéine de liaison de calmoduline, de marqueur substance P, le marqueur RYIRS, le marqueur par épitope d'hémagglutinine A, le marqueur Glu-Glu et le marqueur FLAG.

13. Complexe protéique homotrimérique comprenant un polypeptide, ledit polypeptide comprenant (1) un domaine extracellulaire TACI et (2) un fragment de trimérisation de la protéine de liaison de choc thermique-1 pour inhiber la prolifération des lymphocytes B induite par la TNF4.
